(19) **Europäisches Patentamt European Patent Office Office européen des brevets**

(11) **EP 4 737 582 A1**

(12) **EUROPEAN PATENT APPLICATION**
published in accordance with Art. 153(4) EPC

(43) Date of publication:
06.05.2026 Bulletin 2026/19

(21) Application number: 24831906.3

(22) Date of filing: 24.06.2024

(51) International Patent Classification (IPC):
$C12P\ 19/14^{(2006.01)}$    $A23L\ 5/00^{(2016.01)}$
$A23L\ 29/30^{(2016.01)}$    $C08B\ 30/12^{(2006.01)}$
$C12P\ 19/04^{(2006.01)}$

(52) Cooperative Patent Classification (CPC):
A23L 5/00; A23L 29/30; C08B 30/12; C12P 19/04;
C12P 19/14

(86) International application number:
PCT/JP2024/022819

(87) International publication number:
WO 2025/005045 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.06.2023 JP 2023107181

(71) Applicant: Futamura Kagaku Kabushiki Kaisha
Nakamura-ku
Nagoya-shi
Aichi 450-0002 (JP)

(72) Inventors:
• ITO Yusaku
  Tahara-shi Aichi 441-3401 (JP)
• IWATA Yoshiaki
  Tahara-shi Aichi 441-3401 (JP)

(74) Representative: TBK
Bavariaring 4-6
80336 München (DE)

(54) **GEL-LIKE STARCH PARTIAL DECOMPOSITION PRODUCT AND PROCESSED FOOD**

(57) The present invention provides a gelatinous starch partial decomposition product that is improved in heat resistance and suitable as a raw material for foods to be cooked by heating, and a processed food. A gelatinous starch partial decomposition product that has been made gelatinous using a gelatinizable starch partial decomposition product obtained by decomposing a raw material starch with an enzyme, wherein the gelatinizable starch partial decomposition product has a dextrose equivalent of 1.4 to 3.5, and the gelatinous starch partial decomposition product has a weight average molecular weight of 200,000,000 to 520,000,000, a number average molecular weight of 4,000 to 12,000, and a jelly strength of 900 g to 4,300 g as measured in accordance with JIS K 6503 (2001), and changes in property from gel to liquid above 70°C.

**Description**

Technical Field

The present invention relates to a gelatinous starch partial decomposition product and a processed food, and in particular, relates to a gelatinous starch partial decomposition product that serves as a raw material suitable for imparting a texture of fattiness to processed foods, and a processed food using the gelatinous starch partial decomposition product.

Background Art

For example, in livestock meat processed foods such as sausages, hams, and meatballs, it has been proposed to use dextrins as substitutes for fat for the purpose of calorie reduction and the like (see Patent Literature 1). Dextrins are gelatinizable starch partial decomposition products obtained by hydrolysis of starch, and are known to exhibit properties such as liquid or gel depending on the concentration when dissolved in water and other factors. Therefore, dextrins can be utilized, by taking advantage of these properties, in oil and fat substitute foods and the like, such as those described above.
The gelatinizable starch partial decomposition product can be easily produced by, for example, hydrolyzing starch with an enzyme (see Patent Literature 2). The gelatinizable starch partial decomposition product obtained by such enzymatic hydrolysis can be easily dissolved in water at ordinary temperature, and at the same time, can maintain a good viscosity.
In livestock meat processed foods such as sausages, salamis, meats, and meatballs, a variety of devised foods are marketed, such as livestock meat processed foods softened by cooking by heating in which oils and fats or dairy products are enclosed inside, and foods provided with juicy feeling by the inclusion of oils and fats. However, for example, due to rising raw material prices of dairy products and oils and fats, an increase in food costs is unavoidable, and such rises in raw material prices are occurring in various food materials. Therefore, it is demanded to reduce costs by replacing part of the raw materials of processed foods with substitute foods.
The inventors have diligently studied the utilization of a gelatinous starch partial decomposition product obtained using a gelatinizable starch partial decomposition product as a food intended to be provided as the above-described substitute food for food materials such as oils and fats that are softened by cooking by heating, or as substitutes for people with allergies to oils and fats and the like. Although conventional gelatinous starch partial decomposition products can maintain a moderate viscosity at ordinary temperature, they have difficulty exhibiting properties in which they are softened while keeping a predetermined viscosity, as oils and fats do, under relatively high-temperature conditions such as after cooking by heating.

Citation List

Patent Literature

Patent Literature 1: Japanese Patent No. 3169430
Patent Literature 2: Japanese Patent No. 5164340

Summary of Invention

The present invention has been made in view of the above points, and provides a gelatinous starch partial decomposition product that is improved in heat resistance and suitable as a raw material for foods to be cooked by heating, and a processed food.

Solution to Problem

That is, the first invention relates to a gelatinous starch partial decomposition product that has been made gelatinous using a gelatinizable starch partial decomposition product obtained by decomposing a raw material starch with an enzyme, wherein the gelatinizable starch partial decomposition product has a dextrose equivalent of 1.4 to 3.5, and the gelatinous starch partial decomposition product has a weight average molecular weight of 200,000,000 to 520,000,000, a number average molecular weight of 4,000 to 12,000, and a jelly strength of 900 g to 4,300 g as measured in accordance with JIS K 6503 (2001), and changes in property from gel to liquid above 70°C.
The second invention relates to the gelatinous starch partial decomposition product according to the first invention, wherein the gelatinous starch partial decomposition product has a molecular weight dispersity (weight average molecular weight / number average molecular weight) of 42,000 to 60,000.

The third invention relates to the gelatinous starch partial decomposition product according to the second invention, wherein the gelatinous starch partial decomposition product has a molecular weight dispersity (weight average molecular weight / number average molecular weight) of 46,000 to 58,000.

The fourth invention relates to the gelatinous starch partial decomposition product according to any one of the first to third inventions, wherein the dextrose equivalent is 2.2 to 3.0 and the jelly strength is 960 g to 2,400 g.

The fifth invention relates to the gelatinous starch partial decomposition product according to any one of the first to third inventions, wherein the weight average molecular weight is 220,000,000 to 330,000,000 and the number average molecular weight is 4,300 to 7,000.

The sixth invention relates to the gelatinous starch partial decomposition product according to the fourth invention, wherein the weight average molecular weight is 220,000,000 to 330,000,000 and the number average molecular weight is 4,300 to 7,000.

The seventh invention relates to the gelatinous starch partial decomposition product according to the first invention, wherein the raw material starch is potato starch.

The eighth invention relates to the gelatinous starch partial decomposition product according to the first invention, wherein a storage modulus G1 and a loss modulus G2 of the gelatinous starch partial decomposition product at 40°C to 70°C satisfy a relationship of the following expression (i):

$$G1 - G2 > 0 \quad \ldots \quad (i).$$

The ninth invention relates to the gelatinous starch partial decomposition product according to any one of the first to third, seventh, and eighth inventions, wherein the gelatinous starch partial decomposition product is used for any of the following applications: a viscosity imparting agent for foods, an oil and fat substitute food, and a pseudo-oily and fatty material.

The tenth invention relates to the gelatinous starch partial decomposition product according to the fourth invention, wherein the gelatinous starch partial decomposition product is used for any of the following applications: a viscosity imparting agent for foods, an oil and fat substitute food, and a pseudo-oily and fatty material.

The eleventh invention relates to the gelatinous starch partial decomposition product according to the fifth invention, wherein the gelatinous starch partial decomposition product is used for any of the following applications: a viscosity imparting agent for foods, an oil and fat substitute food, and a pseudo-oily and fatty material.

The twelfth invention relates to the gelatinous starch partial decomposition product according to the sixth invention, wherein the gelatinous starch partial decomposition product is used for any of the following applications: a viscosity imparting agent for foods, an oil and fat substitute food, and a pseudo-oily and fatty material.

The thirteenth invention relates to a processed food using the gelatinous starch partial decomposition product according to the ninth invention.

The fourteenth invention relates to a processed food using the gelatinous starch partial decomposition product according to the tenth invention.

The fifteenth invention relates to a processed food using the gelatinous starch partial decomposition product according to the eleventh invention.

The sixteenth invention relates to a processed food using the gelatinous starch partial decomposition product according to the twelfth invention.

Advantageous Effects of Invention

According to the first invention, a gelatinous starch partial decomposition product that has been made gelatinous using a gelatinizable starch partial decomposition product obtained by decomposing a raw material starch with an enzyme, wherein the gelatinizable starch partial decomposition product has a dextrose equivalent of 1.4 to 3.5, and the gelatinous starch partial decomposition product has a weight average molecular weight of 200,000,000 to 520,000,000, a number average molecular weight of 4,000 to 12,000, and a jelly strength of 900 g to 4,300 g as measured in accordance with JIS K 6503 (2001), and changes in property from gel to liquid above 70°C, heat resistance is improved and its gel property can be maintained until exceeding 70°C. As a result, the gelatinous starch partial decomposition product becomes suitable as a raw material for foods to be cooked by heating.

According to the second invention, the gelatinous starch partial decomposition product according to the first invention, wherein the gelatinous starch partial decomposition product has a molecular weight dispersity (weight average molecular weight / number average molecular weight) of 42,000 to 60,000, the molecular weight dispersity is adjusted to a predetermined value and the heat resistance of the gelatinous starch partial decomposition product can be improved.

According to the third invention, the gelatinous starch partial decomposition product according to the second invention, wherein the gelatinous starch partial decomposition product has a molecular weight dispersity (weight average molecular weight / number average molecular weight) of 46,000 to 58,000, the molecular weight dispersity is adjusted to a predetermined value and the heat resistance of the gelatinous starch partial decomposition product can be improved.

According to the fourth invention, the gelatinous starch partial decomposition product according to any one of the first to third inventions, wherein the dextrose equivalent is 2.2 to 3.0 and the jelly strength is 960 g to 2,400 g, the productivity of the gelatinous starch partial decomposition product is improved.

According to the fifth invention, the gelatinous starch partial decomposition product according to any one of the first to third inventions, wherein the weight average molecular weight is 220,000,000 to 330,000,000 and the number average molecular weight is 4,300 to 7,000, the productivity of the gelatinous starch partial decomposition product is improved.

According to the sixth invention, the gelatinous starch partial decomposition product according to the fourth invention, wherein the weight average molecular weight is 220,000,000 to 330,000,000 and the number average molecular weight is 4,300 to 7,000, the productivity of the gelatinous starch partial decomposition product is improved.

According to the seventh invention, the gelatinous starch partial decomposition product according to the first invention, wherein the raw material starch is potato starch, the raw material is easily obtainable and the cost for obtaining the gelatinous starch partial decomposition product can be reduced.

According to the eighth invention, the gelatinous starch partial decomposition product according to the first invention, satisfying a relational expression in which a value obtained by subtracting a loss modulus G2 from a storage modulus G1 of the gelatinous starch partial decomposition product at 40°C to 70°C is larger than 0, the gelatinous starch partial decomposition product exhibits stronger behavior as an elastic body at 40°C to 70°C and can maintain its gelatinous property well.

According to the ninth invention, the gelatinous starch partial decomposition product according to any one of the first to third, seventh, and eighth inventions, wherein the gelatinous starch partial decomposition product is used for any of the following applications: a viscosity imparting agent for foods, an oil and fat substitute food, and a pseudo-oily and fatty material, the gelatinous starch partial decomposition product can be used as a viscosity imparting agent for foods or the like.

According to the tenth invention, the gelatinous starch partial decomposition product according to the fourth invention, wherein the gelatinous starch partial decomposition product is used for any of the following applications: a viscosity imparting agent for foods, an oil and fat substitute food, and a pseudo-oily and fatty material, the gelatinous starch partial decomposition product can be used as a viscosity imparting agent for foods or the like.

According to the eleventh invention, the gelatinous starch partial decomposition product according to the fifth invention, wherein the gelatinous starch partial decomposition product is used for any of the following applications: a viscosity imparting agent for foods, an oil and fat substitute food, and a pseudo-oily and fatty material, the gelatinous starch partial decomposition product can be used as a viscosity imparting agent for foods or the like.

According to the twelfth invention, the gelatinous starch partial decomposition product according to the sixth invention, wherein the gelatinous starch partial decomposition product is used for any of the following applications: a viscosity imparting agent for foods, an oil and fat substitute food, and a pseudo-oily and fatty material, the gelatinous starch partial decomposition product can be used as a viscosity imparting agent for foods or the like.

According to the thirteenth invention, a processed food using the gelatinous starch partial decomposition product according to the ninth invention, it is possible to provide a processed food to be cooked by heating, in which the gelatinous starch partial decomposition product with improved heat resistance is used as a raw material for the processed food.

According to the fourteenth invention, a processed food using the gelatinous starch partial decomposition product according to the tenth invention, it is possible to provide a processed food to be cooked by heating, in which the gelatinous starch partial decomposition product with improved heat resistance is used as a raw material for the processed food.

According to the fifteenth invention, a processed food using the gelatinous starch partial decomposition product according to the eleventh invention, it is possible to provide a processed food to be cooked by heating, in which the gelatinous starch partial decomposition product with improved heat resistance is used as a raw material for the processed food.

According to the sixteenth invention, a processed food using the gelatinous starch partial decomposition product according to the twelfth invention, it is possible to provide a processed food to be cooked by heating, in which the gelatinous starch partial decomposition product with improved heat resistance is used as a raw material for the processed food.

Brief Description of Drawings

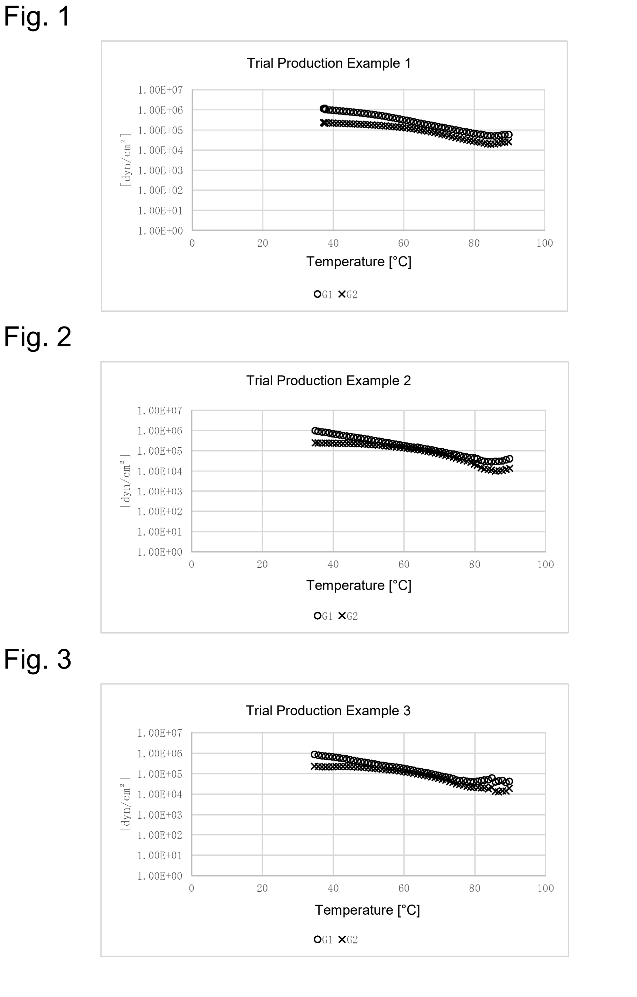

[Figure 1] Figure 1 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 1 gelatinized at a concentration of 40 wt%).

[Figure 2] Figure 2 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 2 gelatinized at a concentration of 40 wt%).

[Figure 3] Figure 3 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 3 gelatinized at a concentration of 40 wt%).

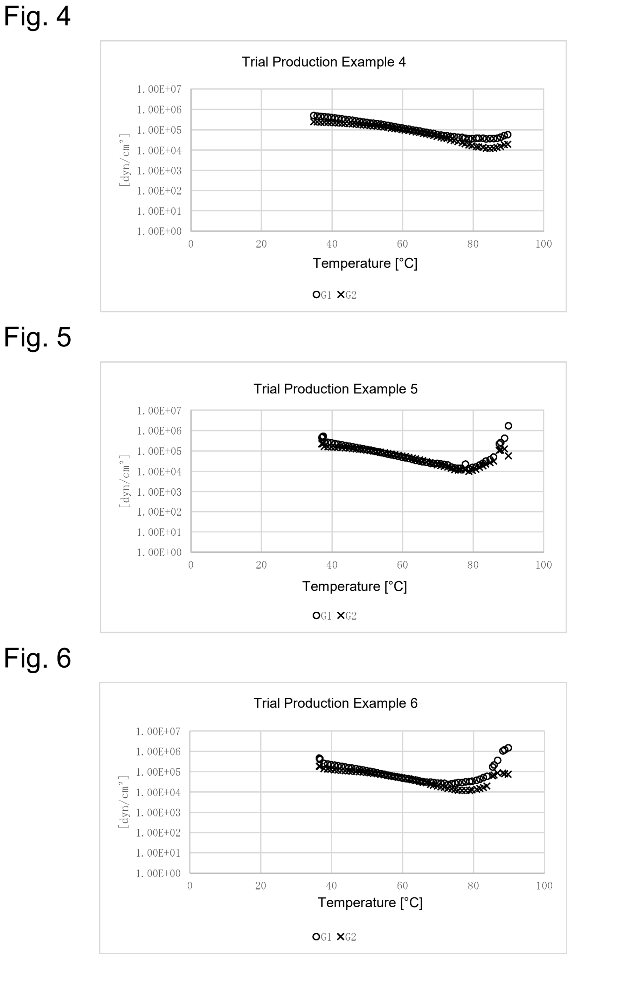

[Figure 4] Figure 4 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 4 gelatinized at a concentration of 40 wt%).

[Figure 5] Figure 5 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 5 gelatinized at a concentration of 40 wt%).

[Figure 6] Figure 6 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 6 gelatinized at a concentration of 40 wt%).

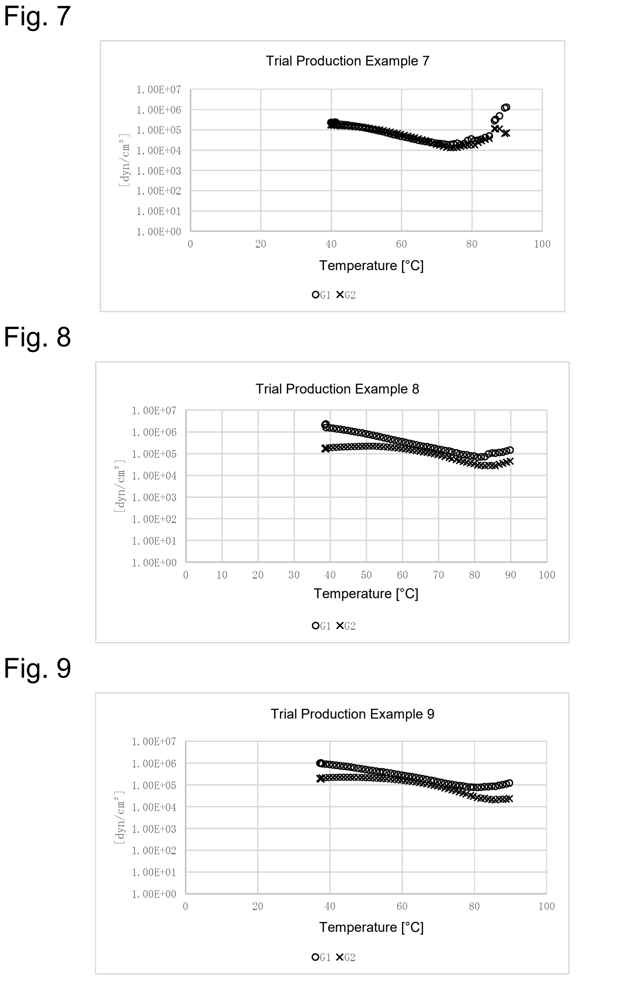

[Figure 7] Figure 7 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 7 gelatinized at a concentration of 40 wt%).

[Figure 8] Figure 8 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 8 gelatinized at a concentration of 40 wt%).

[Figure 9] Figure 9 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 9 gelatinized at a concentration of 40 wt%).

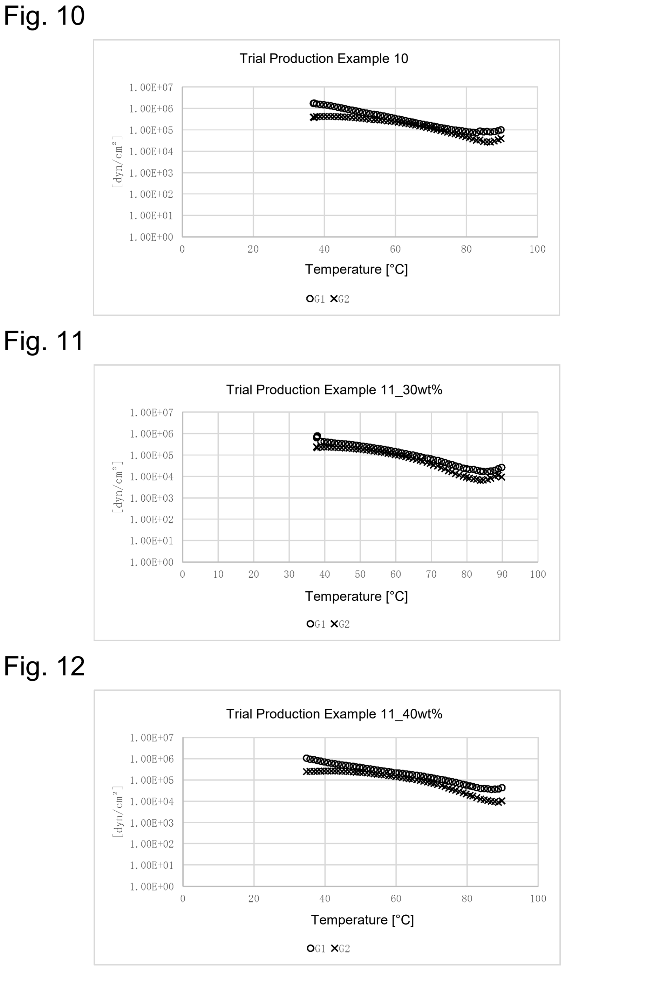

[Figure 10] Figure 10 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 10 gelatinized at a concentration of 40 wt%).

[Figure 11] Figure 11 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 11 gelatinized at a concentration of 30 wt%).

[Figure 12] Figure 12 is a graph showing changes in a storage modulus G1 and a loss modulus G2 with a change in temperature of a gelatinous starch partial decomposition product according to one embodiment of the present invention (the gelatinizable starch partial decomposition product of Trial Production Example 11 gelatinized at a concentration of 40 wt%).

Description of Embodiments

A gelatinous starch partial decomposition product according to one embodiment of the present invention is obtained by gelatinizing a gelatinizable starch partial decomposition product obtained by hydrolyzing a raw material starch with an enzyme, by dissolving it in water or the like. The raw material starch is constituted by amylose, amylopectin, and the like. The amylose is a linear chain in which $\alpha$-D-glucopyranose units are linked through $\alpha$-1,4 bonds, and the amylopectin has a linear portion in which $\alpha$-D-glucopyranose units are linked through $\alpha$-1,4 bonds and a branched portion linked through $\alpha$-1,6 bonds. When the $\alpha$-1,4 bonds of the starch are decomposed with an enzyme, a gelatinizable starch partial decomposition product of dextrins is produced. The dextrins are called dextrin, maltodextrin, maltooligosaccharides, and the like, and are sugar chains having a linear structure or a branched structure.

An enzyme used for decomposition of the starch may be any enzyme as long as it can hydrolyze $\alpha$-1,4 bonds of the starch, and various enzymes such as α-amylase [1,4-α-D-glucan glucanohydrolase (EC 3.2.1.1)] are optimal. Many of these enzymes are derived from the genus Aspergillus, the genus Bacillus, and the like. Of course, from the viewpoint of chemical kinetics, the higher the optimum temperature is, the more desirable it is in order to enhance reactivity. Accordingly, the enzyme used for decomposition of the starch is more preferably an α-amylase derived from thermophilic bacteria of the same genus having an optimum temperature of 70 to 90°C.

The starch serving as a raw material for the gelatinous starch partial decomposition product is not particularly limited, and commercially available, easily obtainable types are used. For example, in addition to starches of corn (corn starch), wheat, barley, rye, rice, sweet potato (sweet potato starch), potato (potato starch), pea, edamame, tapioca, and the like, starches of glutinous species such as glutinous wheat, glutinous millet, and glutinous barnyard millet, as well as waxy corn starch, glutinous rice starch, and the like, can also be used. Among these, the raw material starch is preferably potato (potato starch). Since potato starch is easily obtainable, it is suited as the raw material.

The gelatinizable starch partial decomposition product is obtained by drying a hydrolysis product formed by hydrolyzing starch with an enzyme, and is a product in a dried state such as a powder or solid form. As a drying method carried out after hydrolysis, known drying methods such as a spray drying method (spray drying), a vacuum freeze drying method, and a vacuum drying method with a vacuum drum dryer are used as appropriate. The gelatinizable starch partial decomposition product is gelatinized by dissolving it in water or the like and lowering its temperature to a predetermined temperature as shown in the Examples, thereby forming a gelatinous starch partial decomposition product.

In general, gelatinous starch partial decomposition products are used as substitutes for fat in livestock meat processed foods, and have moderate viscosity at ordinary temperature and liquefy at high temperature. Therefore, it has been difficult to obtain properties in which they are softened while keeping a predetermined viscosity under relatively high-temperature conditions such as after cooking by heating. For this reason, conventional gelatinous starch partial decomposition products were unsuited as substitute foods for food materials that are softened by cooking by heating, such as retort foods. Accordingly, the present inventors have made diligent studies for the purpose of obtaining a gelatinous starch partial decomposition product that exhibits properties of being softened while keeping a predetermined viscosity under relatively high-temperature conditions such as after cooking by heating, as a substitute food for oil and fat food materials such as sausages, hams, and meatballs, or as a substitute food or pseudo-food targeted at calorie reduction in pseudo-oily and fatty materials, margarine, butter, lard, and the like. As a result, it has been found that, among indices for understanding the properties of natural polymer compounds such as starch or resin polymer compounds, by adjusting the dextrose equivalent (DE), the weight average molecular weight (Mw), and the number average molecular weight (Mn) to predetermined value, and further, by setting the jelly strength to 900 g to 4300 g, the gelatinous starch partial decomposition product changes in property from gel to liquid above 70°C. It should be noted that although the present invention mentions foods and retort foods as examples, these are merely examples, and the invention can also be applied to products to be eaten by animals, such as pet foods.

The dextrose equivalent (DE) is one of the indices for understanding the decomposition progress of starch in the gelatinizable starch partial decomposition product. Dextrose is another name for grape sugar (glucose), and starch is a polymer having grape sugar as constituent units. When DE = 0, it indicates that the starch has not been decomposed, and the closer the DE value is to 0, the less the starch is decomposed and the more it exhibits properties close to starch. In general, when DE is 10 or less, it is called dextrin. When DE = 100, it indicates that the starch has been completely decomposed into grape sugar, and the closer the DE value is to 100, the more the starch has been decomposed and the lower the molecular weight has become. The dextrose equivalent is measured by methods such as the Lane-Eynon method, the Bertrand method, and the Willstatter-Schudel method. In the Examples, the Willstatter-Schudel method, which is a common method for quantification of reducing sugars, is used.

The dextrose equivalent (DE) in the gelatinizable starch partial decomposition product of the present invention is set to 1.4 to 3.5, and preferably 2.2 to 3.0. When the dextrose equivalent is too low, the starch partial decomposition product is gelatinized during the production process, making production with general facilities difficult, and when the dextrose equivalent is too high, it becomes difficult to obtain practical gelatinization properties because the amount of polymer responsible for water retention decreases. In addition, the lower the dextrose equivalent (DE), the lower the degree of decomposition of starch in the gelatinizable starch partial decomposition product, and therefore, the viscosity becomes higher. For this reason, the productivity of the gelatinous starch partial decomposition product tends to decrease. On the other hand, the higher the dextrose equivalent (DE), the higher the degree of decomposition of starch in the gelatinizable starch partial decomposition product, and therefore, the viscosity becomes lower. For this reason, the productivity of the gelatinous starch partial decomposition product becomes favorable. When the dextrose equivalent is 1.4 to 3.5, decomposition of starch is relatively mildly suppressed, and therefore, the gelatinizable starch partial decomposition product is easily dissolved in water (liquid) and is excellent in handling convenience.

The weight average molecular weight (Mw) is one of the indices of average molecular weight used when understanding the properties of natural polymer compounds and the like such as starch, and is defined by the following expression (ii). It should be noted that, in the expression (ii), $M_i$ is the molecular weight of a molecule present in the polymer, and $N_i$ is the number of molecules having a molecular weight of $M_i$.

[Expression 1]

$$Mw = \Sigma \ (Mi^2 \ Ni)/\Sigma MiNi \ \ldots \ (ii)$$

The weight average molecular weight (Mw) is, as understood from the expression (ii), highly sensitive to the size of the constituent molecules. Therefore, the influence of a very small amount of high-molecular weight molecules can also be taken into account in the value as an average molecular weight. In the gelatinous starch partial decomposition product of the present invention, the weight average molecular weight (Mw) is 200,000,000 to 520,000,000, preferably 220,000,000 to 330,000,000. When the weight average molecular weight is too small, it becomes difficult to obtain practical gelatinization properties because the amount of polymer responsible for water retention decreases. When the weight average molecular weight is too large, the starch partial decomposition product is gelatinized during the production process, making production with general facilities difficult. The smaller the weight average molecular weight, the more favorable the productivity of the gelatinous starch partial decomposition product tends to be. The larger the weight average molecular weight, the more difficult it becomes to produce the gelatinous starch partial decomposition product with general facilities, and the productivity tends to decrease. By setting the weight average molecular weight to 200,000,000 to 520,000,000, both obtaining practical gelatinization properties and productivity can be achieved. It should be noted that known high performance liquid chromatography is used as a method for measuring the weight average molecular weight.

The number average molecular weight (Mn) is an index representing the average of molecular weight per molecule and is defined by the following expression (iii). In the expression (iii), $M_i$ and $N_i$ are the same as those in the above expression (ii). In the gelatinous starch partial decomposition product of the present invention, the number average molecular weight (Mn) is 4,000 to 12,000, preferably 4,300 to 7,000. When the number average molecular weight is too small, it becomes difficult to obtain practical gelatinization properties because the amount of polymer responsible for water retention decreases. When the number average molecular weight is too large, the starch partial decomposition product is gelatinized during the production process, making production with general facilities difficult. By setting the number average molecular weight (Mn) to 4,000 to 12,000, both obtaining practical gelatinization properties and productivity can be achieved. It should be noted that known high performance liquid chromatography is used as a method for measuring the number average molecular weight.

[Expression 2]

$$Mn = \Sigma MiNi/\Sigma Ni \ \ldots \ (iii)$$

In the gelatinous starch partial decomposition product of the present invention, the jelly strength is 900 g to 4,300 g, more preferably 960 g to 2,400 g. The jelly strength is an index for understanding the hardness of a gelatinous material and is measured in accordance with JIS K 6503 (2001). When the jelly strength is too low, the gel-forming performance is reduced, making it difficult to maintain shape at a high temperature zone. When the jelly strength is too high, it adversely affects the texture of processed foods, making it unsuited as a raw material. By setting the jelly strength to 900 g to 4,300 g, the gelatinous starch partial decomposition product provides good elasticity to processed foods. Furthermore, the gelatinous starch partial decomposition product can secure viscous properties that allow it to be softened while keeping a predetermined viscosity under relatively high-temperature conditions such as after cooking by heating.

In the gelatinous starch partial decomposition product of the present invention, as shown in the Examples described later, it has been found that, among the indices for understanding the properties of natural polymer compounds such as starch or resin polymer compounds, when the dextrose equivalent (DE), the weight average molecular weight (Mw), and the number average molecular weight (Mn) satisfy the above conditions and when the jelly strength satisfies the above conditions, the gelatinous starch partial decomposition product changes in property from gel to liquid above 70°C, preferably at or above 75°C, and more preferably at or above 95°C. In particular, it has been found that the lower the degree of decomposition of the gelatinous starch partial decomposition product, that is, the lower the dextrose equivalent (DE), the higher the temperature at which the property change of the gelatinous starch partial decomposition product occurs tends to be.

Thus, by having the property change of the gelatinous starch partial decomposition product occur at a temperature range above 70°C, the gelatinous starch partial decomposition product exhibits properties of being softened while keeping a predetermined viscosity under relatively high-temperature conditions, such as after cooking by heating of processed foods. Furthermore, the higher the temperature at which the property change occurs, the more slowly the gelatinous starch partial decomposition product is softened. That is, in the gelatinous starch partial decomposition product, the lower the degree of decomposition (DE), the higher the water retention and heat resistance tend to be. Therefore, the gelatinous starch partial decomposition product can be suitably used as a substitute food for food materials that are softened by cooking by heating, such as sausages, hams, meatballs, lard, margarine, and butter. In particular, the gelatinous starch partial decomposition product, which undergoes property change at a high temperature, can be suitably used as a substitute food for foods that are cooked by heating at a relatively high temperature.

Also, in the gelatinous starch partial decomposition product of the present invention, it is preferable that the storage modulus G1 and the loss modulus G2 at 40°C to 70°C satisfy the relationship of the following expression (i) :

$$G1 - G2 > 0 \ldots (i).$$

The storage modulus G1 described above is a value representing the behavior as an elastic body, and the loss modulus G2 is a value representing the behavior as a viscous body. The storage modulus G1 and the loss modulus G2 are measured by dynamic viscoelasticity measurement. When the relationship of the above expression (i) is satisfied and the storage modulus G1 is larger than the loss modulus G2, the gelatinous starch partial decomposition product exhibits stronger behavior as an elastic body and can maintain its gelatinous property well.

Also, in the gelatinous starch partial decomposition product of the present invention, the molecular weight dispersity is 42,000 to 60,000, preferably 46,000 to 58,000. The molecular weight dispersity is an index for understanding the breadth of the molecular weight distribution and is calculated as follows: the weight average molecular weight Mw / the number average molecular weight Mn. When the molecular weight dispersity is too small, the amount of polymer responsible for water retention decreases, making it difficult to obtain practical gelatinization properties, and when the molecular weight dispersity is too large, the starch partial decomposition product is gelatinized during the production process, making production with general facilities difficult. By setting the molecular weight dispersity to 42,000 to 60,000, both obtaining practical gelatinization properties and productivity can be achieved.

In the present invention, the gelatinous starch partial decomposition product is used for any of the following applications: a viscosity imparting agent for foods, an oil and fat substitute food, and a pseudo-oily and fatty material. As a viscosity imparting agent, it is used to adjust the fluidity of liquid-like or paste-like foods. As an oil and fat substitute food or a pseudo-oily and fatty material, it is used in, for example, oil and fat raw materials for livestock meat processed foods such as hams, sausage, meatballs, and lard, or dairy products such as margarine and butter.

Also, the gelatinous starch partial decomposition product of the present invention can be used as a viscosity imparting agent for foods, an oil and fat substitute food, and a pseudo-oily and fatty material, and can be provided as a processed food using the gelatinous starch partial decomposition product of the present invention. Examples of the processed food include foods in which a part of the raw materials of foods such as hams, sausages, meatballs, lard, margarine, and butter have been substituted with the gelatinous starch partial decomposition product, and composite processed foods in which foods such as hams, sausages, meatballs, lard, margarine, and butter have been combined with other processed foods. In these processed foods, since the gelatinous starch partial decomposition product is softened while keeping a predetermined viscosity by cooking by heating, the texture of the above viscosity imparting agent, oil and fat substitute food, and pseudo-oily and fatty material can be favorably reproduced.

Examples

[Processing Treatment of Starch]

An appropriate amount of water was added to commercially available potato starch, α-amylase (manufactured by Amano Enzyme Inc., product number KLEISTASE L1) was added thereto, and enzymatic treatment was carried out using Mini Cooker (manufactured by Noritake Co., Limited, product number NCP-6/10-3/3). After the enzymatic treatment, a liquefied product of the potato starch was subjected to enzyme inactivation treatment, activated carbon treatment, and filtration, and was spray-dried with a spray dryer to obtain gelatinizable starch partial decomposition products of Trial Production Examples 1 to 11.

Next, with respect to the gelatinizable starch partial decomposition products of Trial Production Examples 1 to 11 and the gelatinous starch partial decomposition products obtained therefrom, the following measurement and evaluation methods were carried out. The gelatinous starch partial decomposition products were obtained by diluting the gelatinizable starch partial decomposition products of Trial Production Examples 1 to 11 with water at a predetermined concentration according to each measurement and evaluation method, heating and dissolving to form aqueous starch solutions, and then refrigerating the aqueous starch solutions to gelatinize them. The measurement and evaluation methods performed are for the dextrose equivalent (DE), the weight average molecular weight (Mw), the number average molecular weight (Mn), the molecular weight dispersity (Mw/Mn), the jelly strength, the heat resistance of the gel, the storage modulus and loss modulus, and evaluation of the gel texture. The measurement and evaluation results are shown in Tables 1 to 3 described later.

[Measurement of Dextrose Equivalent]

A powder of 15 g of each gelatinizable starch partial decomposition product of Trial Production Examples 1 to 11 was dissolved in water to prepare 200 ml of an aqueous solution, and the dextrose equivalent (DE) of each gelatinizable starch partial decomposition product of Trial Production Examples 1 to 11 was measured by the Willstatter-Schudel method using the aqueous solution.

[Measurement of Weight Average Molecular Weight, Number Average Molecular Weight, and Molecular Weight Dispersity]

The weight average molecular weight (Mw), the number average molecular weight (Mn), and the molecular weight dispersity (Mw/Mn) were measured based on a HPLC (high performance liquid chromatography) system using a gel permeation chromatography column. The differential refractive index detector used in this HPLC system is RID-20A manufactured by Shimadzu Corporation, the pump is LC-20AD manufactured by Shimadzu Corporation, the column oven is CTO-20A manufactured by Shimadzu Corporation, and the column is SB-806HQ (inner diameter 8 mm $\times$ length 300 mm) manufactured by Resonac Corporation.

The gelatinizable starch partial decomposition products of Trial Production Examples 1 to 11 were diluted and dissolved in pure water to a concentration of 3 wt% (solid concentration), and after filtration using a 0.45 $\mu$m membrane filter, they were loaded into the above HPLC system. Pure water was used as the carrier for separating components in the solution, the flow rate was set to 1.0 mL/min, and the column temperature was set to 70°C. Also, as standard substances with known molecular weights, SHODEX STANDARD P-82 (pullulan; peak top molecular weight 739,000, 334,000, 216,000, 107,000, 49,700, 22,000, 9,800, and 6,300) manufactured by Resonac Corporation was used. Then, the molecular weight distribution data obtained from the HPLC system were analyzed using the chromatogram analysis software SIC $\mu$7 Plus Data Station manufactured by System Instruments Co., Ltd., and the weight average molecular weight (Mw), the number average molecular weight (Mn), and the molecular weight dispersity (Mw/Mn) were calculated. It should be noted that the Z-average molecular weight ($M_z$) in Table 1 is also used as an index for understanding the properties of natural polymer compounds such as starch and resin polymer compounds, and is calculated using the above HPLC system and chromatogram analysis software.

[Measurement of Jelly Strength]

The gelatinizable starch partial decomposition products of Trial Production Examples 1 to 11 were diluted in water to a concentration of 30 wt% (solid concentration), and then heated in a microwave to dissolve them, forming aqueous starch solutions. These aqueous starch solutions were refrigerated at 10°C for 17 $\pm$ 1 hours in accordance with JIS K 6503 (2001) to gelatinize into the form of jelly, thereby obtaining the gelatinous starch partial decomposition products. Thereafter, using Rheometer COMPAC-100II manufactured by Sun Scientific Co., Ltd., the jelly strength of the gelatinous starch partial decomposition products was measured in accordance with JIS K 6503 (2001).

[Evaluation of Heat Resistance of Gel]

The gelatinizable starch partial decomposition products of Trial Production Examples 1 to 11 were diluted in water to a concentration of 40 wt% (solid concentration), and then heated in a microwave to dissolve them, forming aqueous starch solutions. Twenty grams of these aqueous starch solutions were refrigerated at 10°C for 17 hours to gelatinize into the form of jelly, thereby obtaining the gelatinous starch partial decomposition products. It should be noted that, as for the gelatinizable starch partial decomposition product of Trial Production Example 11, an additional aqueous starch solution at a concentration of 30 wt% (solid concentration) was also fabricated by the same method to obtain the gelatinous starch partial decomposition product. Thereafter, the gelatinous starch partial decomposition products were die-cut using a stainless steel jig with a diameter of 35 mm. The resulting gelatinous starch partial decomposition products with a thickness of 3 mm were heated as samples for 1 minute on a hot plate (HPR-4030 manufactured by AS ONE Corporation) that had been heated to a predetermined temperature. After heating, the gelatinous starch partial decomposition products were peeled off from the hot plate, and the degree of stickiness on the hot plate surface at that time was evaluated. The heat resistance evaluation was carried out at temperatures starting from 50°C, increasing by 10°C increments. In the case where no stickiness was observed on the hot plate surface, the sample was evaluated as "Good"; in the case where slight stickiness was observed on the hot plate surface, the sample was evaluated as "Fair"; and in the case where stickiness was observed on the hot plate surface, it was evaluated as "Poor". It should be noted that the heating was stopped at the temperature at which each sample of Trial Production Examples 1 to 10 and Trial Production Example 11 (30 wt%) was evaluated as "Poor", and for Trial Production Example 11 (40 wt%), the heating was stopped at 100°C.

[Measurement of Storage Modulus and Loss Modulus]

The gelatinizable starch partial decomposition products of Trial Production Examples 1 to 10 were diluted in water to a concentration of 40 wt% (solid concentration), and the gelatinizable starch partial decomposition product of Trial Production Example 11 was diluted in water to concentrations of 30 wt% and 40 wt% (solid concentration), and then heated in a microwave to dissolve them, forming aqueous starch solutions. Twenty grams of these aqueous starch solutions were refrigerated at 10°C for 24 hours to gelatinize into the form of jelly, thereby obtaining the gelatinous starch partial decomposition products. After that, the gelatinous starch partial decomposition products were die-cut using a stainless steel jig with a diameter of 35 mm. Using the resulting gelatinous starch partial decomposition products with a thickness of 3 mm as samples, the storage modulus G1 and the loss modulus G2 were measured by using a dynamic viscoelasticity measurement apparatus (Rheosol-G1000T manufactured by UBM Co., Ltd.), and subjecting the samples sandwiched between the cup plate on the lower side and the parallel plate on the upper side to a continuous strain of 1 degree at a frequency of 1 Hz while being heated from 35°C to 90°C at a rate of 1°C/min. Figures 1 to 12 show the measurement results of the storage modulus G1 and the loss modulus G2 of the respective gelatinous starch partial decomposition products corresponding to Trial Production Examples. Then, evaluation was made on whether the storage modulus G1 and the loss modulus G2 measured between 40°C and 70°C always satisfied the relationship G1 - G2 > 0.

[Evaluation of Gel Texture]

The gelatinizable starch partial decomposition products of Trial Production Examples 1 to 11 were dissolved and gelatinized under the same conditions as the evaluation of heat resistance of the gel, thereby obtaining the gelatinous starch partial decomposition products. Thereafter, using Economical Forced Convection Oven DKM600 manufactured by Yamato Scientific Co., Ltd., 2 g of the samples of the gelatinous starch partial decomposition products were heated at 80°C for 1 minute. The gel texture was sensorily evaluated for both the samples after heating at 80°C and the samples stored at ordinary temperature without heating. The sensory evaluation was performed by six panelists, and the elasticity, thickness, and resistance to melting were evaluated by comparison with Trial Production Example 5 as the reference product according to the following criteria.

[Elasticity of Gel]

As for elasticity, the elasticity of the sample was evaluated when it was placed in the mouth and chewed with teeth. The evaluation was performed by the five-point method, in which the reference product, Trial Production Example 5, was set as 3 points, and the samples were scored from 1 point to 5 points by 1 point increments as follows.

   5 points: Significantly more elastic than the reference product.
   4 points: Slightly more elastic than the reference product.
   3 points: Equivalent to the reference product.
   2 points: Slightly softer than the reference product.
   1 point: Significantly softer than the reference product.

[Thickness of Gel]

As for thickness, the tongue feel of the sample was evaluated when it was placed in the mouth and melted. The evaluation was performed by the five-point method in the same manner as the elasticity of the gel, and the evaluation criteria were as follows.

   5 points: Significantly more thickness is felt compared to the reference product.
   4 points: Slightly more thickness is felt compared to the reference product.
   3 points: Equivalent to the reference product.
   2 points: Slightly less thickness is felt compared to the reference product.
   1 point: Significantly less thickness is felt compared to the reference product.

[Resistance to Melting of Gel]

As for resistance to melting, the resistance to melting of the sample in the mouth was evaluated. The evaluation was performed by the five-point method in the same manner as the elasticity and thickness of the gel, and the evaluation criteria were as follows.

   5 points: The sample is able to keep its shape much more than the reference product without melting.
   4 points: The sample is able to keep its shape somewhat more than the reference product without melting.
   3 points: Equivalent to the reference product.

2 points: The sample is somewhat easier to melt than the reference product.
1 point: The sample is much easier to melt than the reference product.

[Table 1]

| Sample | DE | Mw | Mn | Mz | Mw/Mn | Jelly strength [g] |
|---|---|---|---|---|---|---|
| Trial Production Example 1 | 2.0 | 462,163,498 | 7,848 | 935,934,089 | 58,889 | 2,880 |
| Trial Production Example 2 | 2.6 | 260,330,783 | 4,883 | 903,155,008 | 53,314 | 1,410 |
| Trial Production Example 3 | 2.9 | 251,748,292 | 4,397 | 926,075,196 | 57,255 | 1,150 |
| Trial Production Example 4 | 2.9 | 229,437,399 | 4,434 | 891,115,057 | 51,745 | 960 |
| Trial Production Example 5 | 3.6 | 181,583,068 | 3,329 | 926,222,932 | 54,546 | 490 |
| Trial Production Example 6 | 3.6 | 159,467,669 | 3,338 | 919,277,403 | 47,773 | 540 |
| Trial Production Example 7 | 3.6 | 142,651,167 | 3,284 | 904,573,010 | 43,438 | 510 |
| Trial Production Example 8 | 3.0 | 232,947,955 | 4,772 | 940,139,017 | 48,816 | 1,340 |
| Trial Production Example 9 | 2.5 | 305,500,886 | 5,445 | 942,531,430 | 56,107 | 2,360 |
| Trial Production Example 10 | 2.2 | 320,795,459 | 6,854 | 933,089,575 | 46,804 | 2,330 |
| Trial Production Example 11 | 1.4 | 512,563,308 | 11,952 | 944,918,946 | 42,885 | 4,270 |

[Table 2]

| Sample | Concentration (wt%) | Temperature of hot plate | | | | | | Success or failure of G1-G2>0 at 40°C to 70°C |
|---|---|---|---|---|---|---|---|---|
| | | 50 (°C) | 60 (°C) | 70 (°C) | 80 (°C) | 90 (°C) | 100 (°C) | |
| Trial Production Example 1 | 40 | Good | Good | Good | Fair | Poor | - | Good |
| Trial Production Example 2 | 40 | Good | Good | Good | Fair | Poor | - | Good |
| Trial Production Example 3 | 40 | Good | Good | Good | Fair | Poor | - | Good |
| Trial Production Example 4 | 40 | Good | Good | Good | Fair | Poor | - | Good |
| Trial Production Example 5 | 40 | Good | Fair | Poor | - | - | - | Poor |
| Trial Production Example 6 | 40 | Good | Fair | Poor | - | - | - | Poor |
| Trial Production Example 7 | 40 | Good | Fair | Poor | - | - | - | Poor |
| Trial Production Example 8 | 40 | Good | Good | Good | Fair | Poor | - | Good |
| Trial Production Example 9 | 40 | Good | Good | Good | Fair | Poor | - | Good |
| Trial Production Example 10 | 40 | Good | Good | Good | Fair | Poor | - | Good |
| Trial Production Example 11 | 30 | Good | Good | Good | Fair | Poor | - | Good |
| | 40 | Good | Good | Good | Good | Good | Fair | Good |

[Table 3]

| Sample | Concentration (wt%) | Texture (ordinary temperature) | | | Texture (80°C) | | |
|---|---|---|---|---|---|---|---|
| | | Elasticity | Thickness | Resistance to melting | Elasticity | Thickness | Resistance to melting |
| Trial Production Example 1 | 40 | 4.0 | 4.3 | 4.3 | 4.0 | 4.3 | 4.2 |
| Trial Production Example 2 | 40 | 4.0 | 4.0 | 4.0 | 4.0 | 4.2 | 4.0 |
| Trial Production Example 3 | 40 | 3.8 | 4.0 | 3.8 | 3.8 | 4.0 | 4.0 |
| Trial Production Example 4 | 40 | 3.7 | 4.3 | 4.2 | 4.0 | 4.3 | 4.2 |
| Trial Production Example 5 | 40 | 3.0 | 3.0 | 3.0 | 3.0 | 3.0 | 3.0 |
| Trial Production Example 6 | 40 | 3.0 | 3.5 | 3.7 | 3.0 | 3.2 | 3.3 |
| Trial Production Example 7 | 40 | 3.3 | 3.7 | 4.0 | 2.7 | 2.7 | 3.5 |
| Trial Production Example 8 | 40 | 3.3 | 3.7 | 3.7 | 3.8 | 3.7 | 3.7 |
| Trial Production Example 9 | 40 | 4.0 | 3.7 | 4.2 | 4.2 | 3.7 | 3.7 |
| Trial Production Example 10 | 40 | 4.0 | 3.8 | 4.0 | 4.3 | 3.7 | 3.7 |
| Trial Production Example 11 | 30 | 3.2 | 3.5 | 3.2 | 3.3 | 3.3 | 3.2 |
| | 40 | 4.2 | 4.3 | 4.3 | 4.0 | 4.3 | 4.3 |

[Results and Discussion]

In consideration of use as a raw material for processed foods to be cooked by heating, the relationship between the heat resistance of the gel and the storage modulus G1 and the loss modulus G2 was studied. The gelatinous starch partial decomposition products using the gelatinizable starch partial decomposition products of Trial Production Examples 1 to 4 and 8 to 11 had all of the desired performance. In contrast, the gelatinous starch partial decomposition products using the gelatinizable starch partial decomposition products of Trial Production Examples 5 to 7 did not have all of the desired performance.

Therefore, the physical properties of Trial Production Examples 1 to 4 and 8 to 11 were compared with those of Trial Production Examples 5 to 7. No particular differences were observed in the molecular weight dispersity (Mw/Mn) and the Z-average molecular weight ($M_z$), but clear differences were observed in the dextrose equivalent (DE), the weight average molecular weight (Mw), the number average molecular weight (Mn), and the jelly strength. Thus, it is considered that the four indices, dextrose equivalent (DE), weight average molecular weight (Mw), number average molecular weight (Mn), and jelly strength, can be used as indices indicating the physical properties of gelatinous starch partial decomposition products that satisfy the relationship between the heat resistance of the gel and the storage modulus G1 and the loss modulus G2.

From the comparison of the physical properties of Trial Production Examples 1 to 4 and 8 to 11 with those of Trial Production Examples 5 to 7, the following can be derived. The preferable physical properties of the gelatinizable starch partial decomposition products are considered to satisfy all of the following: a dextrose equivalent (DE) of 1.4 to 3.5, a weight average molecular weight (Mw) of 200,000,000 to 520,000,000, a number average molecular weight (Mn) of 4,000 to 12,000, and a jelly strength of 900 g to 4,300 g.

From the comparison of the physical properties of Trial Production Examples 1 to 4 and 8 to 11 with those of Trial Production Examples 5 to 7, the following can also be derived. When the above four indices (DE, Mw, Mn, and jelly strength) are satisfied and the molecular weight dispersity (Mw/Mn) is about 42,000 to 60,000, it is considered that heat resistance is improved and suitable use as a raw material for processed foods to be cooked by heating is enabled, as in the case of the gelatinous starch partial decomposition products corresponding to Trial Production Examples 1 to 4 and 8 to 11.

It should be noted that, for the gelatinous starch partial decomposition products corresponding to Trial Production Examples 1 to 4 and 8 to 11, the following was confirmed. Compared to the gelatinous starch partial decomposition product of Trial Production Example 5 (reference product), the gelatinous starch partial decomposition products corresponding to Trial Production Examples 1 to 4 and 8 to 11 were confirmed, based on the evaluation of the elasticity of the gel, to resist liquefaction and to be able to keep their shape both when stored at ordinary temperature and after heating at 80°C. Furthermore, the gelatinous starch partial decomposition products corresponding to Trial Production Examples 1 to 4 and 8 to 11 were confirmed, based on the evaluation of thickness, to retain thickness and have a superior tongue feel even at a higher temperature (80°C) compared to Trial Production Example 5 (reference product). The gelatinous starch partial decomposition products corresponding to Trial Production Examples 1 to 4 and 8 to 11 were confirmed, based on the evaluation of resistance to melting, to resist melting and tend to keep their shape even at a higher temperature (80°C) compared to Trial Production Example 5 (reference product). In addition, it was confirmed that Trial Production Example 11 has properties where it tends to keep its shape and is softened more slowly even at a higher temperature range (about 100°C).

Accordingly, the gelatinous starch partial decomposition products corresponding to Trial Production Examples 1 to 4 and 8 to 11 were confirmed to have effects equal to or greater than Trial Production Example 5 (reference product) in terms of gel texture (elasticity, thickness, and resistance to melting). As a result, when the gelatinous starch partial decomposition products corresponding to Trial Production Examples 1 to 4 and 8 to 11 are used as raw materials for processed foods, it is considered that processed foods with superior texture, such as elasticity, thickness, and resistance to melting, can be obtained. In particular, in the case of Trial Production Example 11, where the property change occurs at an even higher temperature range (about 100°C), appropriate properties are likely to be obtained even under use situations such as high-temperature cooking like oven cooking, making it suitable as a raw material for processed foods to be cooked by heating at high-temperature.

Also, in the gelatinous starch partial decomposition products corresponding to Trial Production Examples 1 to 4 and 8 to 11, it was observed that the higher the dextrose equivalent (DE) and the lower the weight average molecular weight (Mw), the higher the degree of starch decomposition, making the gelatinization of the gelatinous starch partial decomposition products more difficult, and the productivity tended to be easily increased. From this, in view of productivity, the gelatinous starch partial decomposition products of Trial Production Examples 2 to 4 and 8 to 10 are considered preferable. For this reason, it is considered that, as physical properties corresponding to good productivity of the gelatinizable starch partial decomposition products, a dextrose equivalent (DE) of 2.2 to 3.0 and a jelly strength of 960 g to 2,400 g can be mentioned. In addition, it is considered that, as physical properties corresponding to good productivity of the gelatinous starch partial decomposition products, a weight average molecular weight (Mw) of 220,000,000 to 330,000,000 and a number average molecular weight (Mn) of 4,300 to 7,000 can also be mentioned.

From the above, in the present invention, the gelatinizable starch partial decomposition products have a dextrose equivalent of 1.4 to 3.5, and the gelatinous starch partial decomposition products have a weight average molecular weight of 200,000,000 to 520,000,000, a number average molecular weight of 4,000 to 12,000, and a jelly strength of 900 g to 4,300 g as measured in accordance with JIS K 6503 (2001), and changes in property from gel to liquid above 70°C. Accordingly, heat resistance is improved, and the gel property can be maintained until exceeding 70°C. As a result, the gelatinous starch partial decomposition products can exhibit properties in which they are softened while keeping a predetermined viscosity even under relatively high-temperature conditions such as after cooking by heating, as in sausages, hams, meatballs, lard, margarine, or butter, making them suitable as raw materials for foods to be cooked by heating.

Industrial Applicability

The gelatinous starch partial decomposition product of the present invention has improved heat resistance and can maintain a gel property until exceeding 70°C. For this reason, the gelatinous starch partial decomposition product of the present invention can gradually change its properties during cooking by heating and is suitable as a raw material for foods to be cooked by heating. Accordingly, the gelatinous starch partial decomposition product of the present invention is promising as a new substitute food for food materials that are softened by cooking by heating, such as sausages, hams, meatballs, lard, margarine, and butter.

**Claims**

1. A gelatinous starch partial decomposition product that has been made gelatinous using a gelatinizable starch partial decomposition product obtained by decomposing a raw material starch with an enzyme,

   wherein the gelatinizable starch partial decomposition product has a dextrose equivalent of 1.4 to 3.5, and the gelatinous starch partial decomposition product has a weight average molecular weight of 200,000,000 to 520,000,000, a number average molecular weight of 4,000 to 12,000, and a jelly strength of 900 g to 4,300 g as measured in accordance with JIS K 6503 (2001), and changes in property from gel to liquid above 70°C.

2. The gelatinous starch partial decomposition product according to claim 1, wherein the gelatinous starch partial decomposition product has a molecular weight dispersity (weight average molecular weight / number average molecular weight) of 42,000 to 60,000.

3. The gelatinous starch partial decomposition product according to claim 2, wherein the gelatinous starch partial decomposition product has a molecular weight dispersity (weight average molecular weight / number average molecular weight) of 46,000 to 58,000.

4. The gelatinous starch partial decomposition product according to any one of claims 1 to 3, wherein the dextrose equivalent is 2.2 to 3.0 and the jelly strength is 960 g to 2,400 g.

5. The gelatinous starch partial decomposition product according to any one of claims 1 to 3, wherein the weight average molecular weight is 220,000,000 to 330,000,000 and the number average molecular weight is 4,300 to 7,000.

6. The gelatinous starch partial decomposition product according to claim 4, wherein the weight average molecular weight is 220,000,000 to 330,000,000 and the number average molecular weight is 4,300 to 7,000.

7. The gelatinous starch partial decomposition product according to claim 1, wherein the raw material starch is potato starch.

8. The gelatinous starch partial decomposition product according to claim 1, wherein a storage modulus G1 and a loss modulus G2 of the gelatinous starch partial decomposition product at 40°C to 70°C satisfy a relationship of the following expression (i):

$$G1 - G2 > 0 \ldots (i).$$

9. The gelatinous starch partial decomposition product according to any one of claims 1 to 3, 7, and 8, wherein the gelatinous starch partial decomposition product is used for any of the following applications: a viscosity imparting agent for foods, an oil and fat substitute food, and a pseudo-oily and fatty material.

10. The gelatinous starch partial decomposition product according to claim 4, wherein the gelatinous starch partial decomposition product is used for any of the following applications: a viscosity imparting agent for foods, an oil and fat substitute food, and a pseudo-oily and fatty material.

11. The gelatinous starch partial decomposition product according to claim 5, wherein the gelatinous starch partial decomposition product is used for any of the following applications: a viscosity imparting agent for foods, an oil and fat substitute food, and a pseudo-oily and fatty material.

12. The gelatinous starch partial decomposition product according to claim 6, wherein the gelatinous starch partial decomposition product is used for any of the following applications: a viscosity imparting agent for foods, an oil and fat substitute food, and a pseudo-oily and fatty material.

**13.** A processed food using the gelatinous starch partial decomposition product according to claim 9.

**14.** A processed food using the gelatinous starch partial decomposition product according to claim 10.

**15.** A processed food using the gelatinous starch partial decomposition product according to claim 11.

**16.** A processed food using the gelatinous starch partial decomposition product according to claim 12.

## Fig. 1

Trial Production Example 1

## Fig. 2

Trial Production Example 2

## Fig. 3

Trial Production Example 3

## Fig. 4

Trial Production Example 4

## Fig. 5

Trial Production Example 5

## Fig. 6

Trial Production Example 6

Fig. 7

Trial Production Example 7

Fig. 8

Trial Production Example 8

Fig. 9

Trial Production Example 9

Fig. 10

Trial Production Example 10

Fig. 11

Trial Production Example 11_30wt%

Fig. 12

Trial Production Example 11_40wt%

## INTERNATIONAL SEARCH REPORT

| International application No. |
|---|
| **PCT/JP2024/022819** |

| A. | CLASSIFICATION OF SUBJECT MATTER |
|---|---|

*C12P 19/14*(2006.01)i; *A23L 5/00*(2016.01)i; *A23L 29/30*(2016.01)i; *C08B 30/12*(2006.01)i; *C12P 19/04*(2006.01)i
FI:   C12P19/14 Z; A23L5/00 J; A23L29/30; C08B30/12; C12P19/04

According to International Patent Classification (IPC) or to both national classification and IPC

| B. | FIELDS SEARCHED |
|---|---|

Minimum documentation searched (classification system followed by classification symbols)

C12P19/14; A23L5/00; A23L29/30; C08B30/12; C12P19/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2020-188695 A (FUTAMURA CHEMICAL CO., LTD.) 26 November 2020 (2020-11-26) paragraph [0043] | 1-16 |
| X | JP 2018-143173 A (FUTAMURA CHEMICAL CO., LTD.) 20 September 2018 (2018-09-20) paragraph [0049] | 1-16 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 August 2024** | **03 September 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

| International application No. |
| --- |
| **PCT/JP2024/022819** |

| Patent document cited in search report | | | Publication date (day/month/year) | Patent family member(s) | Publication date (day/month/year) |
| --- | --- | --- | --- | --- | --- |
| JP | 2020-188695 | A | 26 November 2020 | (Family: none) | |
| JP | 2018-143173 | A | 20 September 2018 | (Family: none) | |

Form PCT/ISA/210 (patent family annex) (July 2022)

**REFERENCES CITED IN THE DESCRIPTION**

**Patent documents cited in the description**

- JP 3169430 B
- JP 5164340 B